# EUROPEAN PATENT SPECIFICATION

(11) **EP 2 324 053 B1**
(45) Date of publication and mention of the grant of the patent: **12.08.2015**
(21) Application number: 09768811.3
(22) Date of filing: 18.06.2009
(51) Int. Cl.: C07K 14/435, A61K 39/00

(54) **FERRITIN 2 FOR THE HOST IMMUNIZATION AGAINST TICKS**
FERRITIN-2 ZUR IMMUNISIERUNG VON WIRTEN GEGEN ZECKEN
FERRITINE 2 DESTINÉE À L'IMMUNISATION D'UN HÔTE CONTRE LES TIQUES

(30) Priority: 25.06.2008 CZ 20080402
(43) Date of publication of application: 25.05.2011
(73) Proprietor: Biology Centre As Cr, V.v.i., 37005 Ceske Budejovice (CZ)
(72) Inventor: KOPACEK, Petr, 373 11 Ledenice (CZ); HAJDUSEK, Ondrej, 370 05 Ceske Budejovice (CZ)
(74) Representative: Beetz & Partner mbB
(86) International application number: PCT/CZ2009/000085
(87) International publication number: WO 2009/155886

(56) References cited:
- DATABASE EMBL [Online] 20 April 2008 (2008-04-20), "Ixodes scapularis 1108462734296 genomic scaffold, whole genome shotgun sequence." XP002562989 retrieved from EBI accession no. EMBL:DS954460 Database accession no. DS954460
- HAJDUSEK ONDREJ ET AL: "Knockdown of proteins involved in iron metabolism limits tick reproduction and development." PROCEEDINGS OF THE NATIONAL ACADEMY OF SCIENCES OF THE UNITED STATES OF AMERICA 27 JAN 2009, vol. 106, no. 4, 27 January 2009 (2009-01-27), pages 1033-1038, XP002562990 ISSN: 1091-6490
- KOPÁCEK PETR ET AL: "Molecular cloning, expression and isolation of ferritins from two tick species--Ornithodoros moubata and Ixodes ricinus." January 2003 (2003-01), INSECT BIOCHEMISTRY AND MOLECULAR BIOLOGY JAN 2003, VOL. 33, NR. 1, PAGE(S) 103 - 113 , XP002562991 ISSN: 0965-1748 the whole document
- NICHOL HELEN ET AL: "Iron metabolism in insects." ANNUAL REVIEW OF ENTOMOLOGY 2002, vol. 47, 2002, pages 535-559, XP002562992 ISSN: 0066-4170

## Description

### Technical field

A protein antigen for immunization of the organism - the produced antibody will block an iron transport in the tick body and hereby prevent its long-lasting feeding on the host, eventually block transmission of pathogens from the tick to the host.

### Background art

Untill now, only intracellular ferritin 1 has been known in ticks, which was isolated, cloned and sequenced in our laboratory for the first time from two tick species - Ixodes ricinus and Ornithodoros moubata. Later on, other orthologs of ferritin 1 were sequenced and partially characterized from other tick species in different laboratories. Tick ferritin 1 is post-trascriptionally regulated by the IRP (iron-regulatory protein), which binds IRE (iron-responsive element) loop of the particular mRNA. Ferritin 1 is an oligomer (composed of 24 subunits) and functions in the intracellular iron storage and prevention of the undesirable oxidative effects caused by the free iron.

Database EMBL-EBI [Online] of 20 April 2008 "Ixodes scapularis 1108462734296 genomic scaffold, whole genome shotgun sequence" XP002562989 retrieved from EBPI accession no. EMBL:DS954460 Database accession no. DS954460" discloses the whole genome shotgun sequence of Ixodes scapularis comprising 87576 bp.

P. Kopacek et al. (Insect Biochem. Molec. Biol. 33 (2003) p. 103-113) describe the isolation and cloning of genes encoding ferritins from cDNA libraries of hard tick Ixodes ricinus and soft tick Ornithodoros moubata. The ferritins under investigation are ferritins 1 which are intracellular ferritins having the function of iron storage.

### Disclosure of the invention

Ferritin 2 is a newly identified and characterized protein, which is secreted into the tick plasma. It has been identified in ticks Ixodes ricinus, Ixodes scapularis, Dermacentor variabilis, and Rhipicephalus microplus (formerly Boophilus microplus). We have sequenced these ferritins and determined their primary structures. Ferritin 2 functions in the- tick body as a transporter of iron, which is obtained from the host blood (plasma). When we succeed to block the protein, the attached tick will not be able to feed on the host, because the mechanism of non-heme iron transport from the gut to the peripheral tissue will not be functional, which is essential for tick parasitic life. The blocking of ferritin 2 can be achieved by the reaction with the specific antibody, what leads to the formation of immunocomplex. Specific antibodies will be raised up in the host organism after immunization with the tick antigen ferritin 2. A recombinant ferritin 2 or its fragment expressed in the prokaryotic (*Escherichia coli,* etc.), or eukaryotic (*Pichia pastoris,* insect cells, etc.) expression systems can be used as a suitable antigen for the immunization. Expressed and purified recombinant ferritin 2 could be used for immunization with the appropriate adjuvants or formulation media. The advantage of use of ferritin 2 is its substantial amino-acid sequence difference from the mammalian heavy (H) or light (L) chain ferritins. This substantially reduces a risk of undesirable side effects of the vaccine after the host immunization.

Ticks with decreased synthesis of ferritin 2, caused by RNA interference, are not able to finish their feeding and usually dry and die directly on the host. This fact makes ferritin 2 an ideal candidate for development of vaccine or other compound that will interfere with ferritin 2. Disruption of the tick feeding on the immunized host can impair the normal tick development and possibly will lead to the elimination of pathogen transmission by ticks (e.g., Lyme disease spirochetes), which are dependent on the successful tick feeding.

### Figure

Figure 1: amino acid sequences of Ferritin 2

### Examples

### Example 1

Ferritin 2, designated as FER2-IR, is a protein produced by the tick *Ixodes ricinus* and its primary structure is determined by the amino-acid sequence:

MKQFVVILALIGAATSGNNLFENLDKYPLQDECQAALQEHINVEMHASLVYMQMAAHFDNNKVARKGFSTFFAEN
SKEEREHAQKIIDYINKRGSTVSLVNIDMPLITTWKSVLQALRDAISLENKVTNKLHAVHKIADEECKDPQLMDF
IESEFLEEQVNSIDKLQRMITVLSNMDSGTGEYLLDRELLGDKKEF.

The signal sequence responsible for the protein secretion of the cell is marked in bold. The primary sequence enables correct folding of the protein to the quarter structure, which transports the non-heme iron from the tick gut to the peripheral tissues. In the absence of ferritin 2, or in the case of its successful blocking, the ticks are not able to finish feeding and dry directly on the host.

### Example 2

Ferritin 2, designated as FER2-IS, is a protein produced by the tick *Ixodes scapularis* and its primary structure is determined by the amino-acid sequence.

MKQFVVLLALIGAATSGNNLFENLDKYPLQDECQAALQEHINVEMHASLVYMQMAAHFDNNKVARKGFSTFFAEN
SKEEREHAQKIIDYINKRGSTVSLVNIDMPQITTWKSVLQALRDAISLENKVTNKLHAVHKTADEECKDPQLMDF
IESEFLEEQVTSIDKLQRMITVLSNMDSGTGEYLLDRELLGDKKEF.

The signal sequence responsible for the protein secretion of the cell is marked in bold. The primary sequence enables correct folding of the protein to the quarter structure, which transports the non-heme iron from the tick gut to the peripheral tissues. In the absence of ferritin 2, or in the case of its successful blocking, the ticks are not able to finish feeding and dry directly on the host.

### Example 3

Ferritin 2, designated as FER2-RM, is a protein produced by the tick *Rhipicephalus microplus* and its primary structure is determined by the amino-acid sequence:

MLRIVLVVLAASVALAGNNLNDQVNKYILPDRCRAGLQEQLNELHASLVYMQMAAHLANNKVARGGFARFFRDQ
SSEEREHAQKIIDYLNLRGGTVSAVNVDMPPTAIWMSVLDALQAALALEHRVTNRLYELHRLAEEYDAQMADFLE
QEFLAEQVRSIDQLQRLITQLQNMETGLGEFLLDQQLRA.

The signal sequence responsible for the protein secretion of the cell is marked in bold. The primary sequence enables correct folding of the protein to the quarter structure, which transports the non-heme iron from the tick gut to the peripheral tissues. In the absence of ferritin 2, or in the case of its successful blocking, the ticks are not able to finish feeding and dry directly on the host.

### Example 4

Ferritin 2, designated as FER2-DV, is a protein produced by the tick *Dermacentor variabilis* and its primary structure is determined by the amino-acid sequence:

MLRLALLIALASAAWAGNNLNEQVNQNRYFLHDRCRIGLQEQVNLELHASLVYMQMAAHLANNKVARNGFARFFR
DQSSEEREHAQKLVDYVNLRGGTVSAVSVDMPATATWMSVLDALQAALALEHRVTNRLHELHRLADDSQDPQMAD
FLEQEFLAEQVRSIDQLQRLITQLQNMDTGLGEFLLDQQLRA.

The signal sequence responsible for the protein secretion of the cell is marked in bold. The primary sequence enables correct folding of the protein to the quarter structure, which transports the non-heme iron from the tick gut to the peripheral tissues. In the absence of ferritin 2, or in the case of its successful blocking, the ticks are not able to finish feeding and dry directly on the host.

### Industrial applicability

Production of recombinant ferritin 2 and its use as an antigen for the host immunization against tick feeding and transmission of the tick-borne pathogens.

### Sequence listing part of description

FER2-IR *z Ixodes ricinus* without the signal sequence 
GNNLFENLDKYPLQDECQAALQEHINVEMHASLVYMQMAAHFDNNKVARKGFSTFFAENSKEEREHAQKIIDYIN
KRGSTVSLVNIDMPLITTWKSVLQALRDAISLENKVTNKLHAVHKIADEECKDPQLMDFIESEFLEEQVNSIDKL
QRMITVLSNMDSGTGEYLLDRELLGDKKEF.
FER2-IS z *Ixodes scapularis* 
GNNLFENLDKYPLQDECQAALQEHINVEMHASLVYMQMAAHFDNNKVARKGFSTFFAENSKEEREHAQKIIDYIN
KRGSTVSLVNIDMPQITTWKSVLQALRDAISLENKVTNKLHAVHKTADEECKDPQLMDFIESEFLEEQVTSIDKL
QRMITVLSNMDSGTGEYLLDRELLGDKKEF.
FER2-RM z *Rhipicephalus microplus* 
GNNLNDQVNKYILPDRCRAGLQEQLNLELHASLVYMQMAAHLANNKVARGGFARFFRDQSSEEREHAQKIIDYLN
LRGGTVSAVNVDMPPTAIWMSVLDALQAALALEHRVTNRLYELHRLAEEYDAQMADFLEQEFLAEQVRSIDQLQR
LITQLQNMETGLGEFLLDQQLRA.
FER2-DV z *Dermacentor variabilis*

GNNLNEQVNQNRYFLHDRCRIGLQEQVNLELHASLVYMQMAAHLANNKVARNGFARFFRDQSSEEREHAQKLV
DYVNLRGGTVSAVSVDMPATATWMSVLDALQAALALEHRVTNRLHELHRLADDSQDPQMADFLEQEFLAEQVRSI
DQLQRLITQLQNMDTGLGEFLLDQQLRA

## Claims

1. Ferritin 2 for the host immunization against ticks which has the amino-acid sequence without the signal sequence in at least 85 % identical to the amino-acid sequence of the ferritin 2 of *Ixodes ricinus* (FER2-IR) 
GNNLFENLDKYPLQDECQAALQEHINVEMHASLVYMQMAAHFDNNKVARKGFSTFFAENSK
EEREHAQKIIDYINKRGSTVSLVNIDMPLITTWKSVLQALRDAISLENKVTNKLHAVHKIA
DEECKDPQLMDFIESEFLEEQVNSIDKLQRMITVLSNMDSGTGEYLLDRELLGDKKEF
 or the ferritin 2 of *Ixodes scapularis* (FER2-IS) 
GNNLFENLDKYPLQDECQAALQEHINVEMHASLVYMQMAAHFDNNKVARKGFSTFFAENSK
EEREHAQKIIDYINKRGSTVSLVNIDMPQITTWKSVLQALRDAISLENKVTNKLHAVHKTA
DEECKDPQLMDFIESEFLEEQVTSIDKLQRMITVLSNMDSGTGEYLLDRELLGDKKEF
 or the ferritin 2 of *Rhipicephalus microplus* (FER2-RM) 
GNNLNDQVNKYILPDRCRAGLQEQLNLELHASLVYMQMAAHLANNKVARGGFARFFRDQSS
EEREHAQKIIDYLNLRGGTVSAVNVDMPPTAIWMSVLDALQAALALEHRVTNRLYELHRLA
EEYDAQMADFLEQEFLAEQVRSIDQLQRLITQLQNMETGLGEFLLDQQLRA
 or the ferritin 2 of *Dermacentor variabilis* (FER2-DV) 
GNNLNEQVNQNRYFLHDRCRIGLQEQVNLELHASLVYMQMAAHLANNKVARNGFARFFRDQ
SSEEREHAQKLVDYVNLRGGTVSAVSVDMPATATWMSVLDALQAALALEHRVTNRLHELHR
LADDSQDPQMADFLEQEFLAEQVRSIDQLQRLITQLQNMDTGLGEFLLDQQLRA
 and in the native conditions, it forms the quarter structure, which transports the non-heme iron from the tick gut to the peripheral tissues.

2. Ferritin 2 for the host immunization against ticks according to the claim 1, which has the primary structure without signal sequence composed of amino-acid sequence of the ferritin 2 of *Ixodes ricinus* (FER2-IR).

3. Ferritin 2 for the host immunization against ticks according to the claim 1, which has the primary structure without signal sequence composed of amino-acid sequence of the ferritin 2 of *Ixodes scapularis* (FER2-IS).

4. Ferritin 2 for the host immunization against ticks according to the claim 1, which has the primary structure without signal sequence composed of amino-acid sequence of the ferritin 2 of *Rhipicephalus microplus* (FER2-RM).

5. Ferritin 2 for the host immunization against ticks according to the claim 1, which has the primary structure without signal sequence composed of amino-acid sequence of the ferritin 2 of *Dermacentor variabilis* (FER2-DV).

## Patentansprüche

1. Ferritin 2 zur Immunisierung des Körpers gegen Zecken, dessen Aminosäuresequenz ohne Signalsequenz zumindest zu 85 % identisch ist mit der Aminosäuresequenz
des Ferritins 2 von *Ixodes ricinus* (FER2-IR) 
GNNLFENLDKYPLQDECQAALQEHINVEMHASLVYMQMAAHFDNNKVARKGFSTFFAENSK
EEREHAQKIIDYINKRGSTVSLVNIDMPLITTWKSVLQALRDAISLENKVTNKLHAVHKIA
DEECKDPQLMDFIESEFLEEQVNSIDKLQRMITVLSNMDSGTGEYLLDRELLGDKKEF
 oder des Ferritins 2 von *Ixodes scapularis* (FER2-IS) 
GNNLFENLDKYPLQDECQAALQEHINVEMHASLVYMQMAAHFDNNKVARKGFSTFFAENSK
EEREHAQKIIDYINKRGSTVSLVNIDMPQITTWKSVLQALRDAISLENKVTNKLHAVHKTA
DEECKDPQLMDFIESEFLEEQVTSIDKLQRMITVLSNMDSGTGEYLLDRELLGDKKEF
 oder des Ferritins 2 von *Rhipicephalus microplus* (FER2-RM) 
GNNLNDQVNKYILPDRCRAGLQEQLNLELHASLVYMQMAAHLANNKVARGGFARFFRDQSS
EEREHAQKIIDYLNLRGGTVSAVNVDMPPTAIWMSVLDALQAALALEHRVTNRLYELHRLA
EEYDAQMADFLEQEFLAEQVRSIDQLQRLITQLQNMETGLGEFLLDQQLRA
 oder des Ferritins 2 von *Dermacentor variabilis* (FER2-DV) 
GNNLNEQVNQNRYFLHDRCRIGLQEQVNLELHASLVYMQMAAHLANNKVARNGFARFFRDQ
SSEEREHAQKLVDYVNLRGGTVSAVSVDMPATATWMSVLDALQAALALEHRVTNRLHELHR
LADDSQDPQMADFLEQEFLAEQVRSIDQLQRLITQLQNMDTGLGEFLLDQQLRA
 und das unter nativen Bedingungen eine Quartärstruktur bildet, die das Nicht-Häm-Eisen aus dem Darm der Zecke in periphere Gewebe transportiert.

2. Ferritin 2 für die Immunisierung des Körpers gegen Zecken nach Anspruch 1, dessen Primärstruktur ohne Signalsequenz aus der Aminosäuresequenz des Ferritins 2 von *Ixodes ricinus* (FER2-IR) besteht.

3. Ferritin 2 für die Immunisierung des Körpers gegen Zecken nach Anspruch 1, dessen Primärstruktur ohne Signalsequenz aus der Aminosäuresequenz des Ferritins 2 von *Ixodes scapularis* (FER2-IS) besteht.

4. Ferritin 2 für die Immunisierung des Körpers gegen Zecken nach Anspruch 1, dessen Primärstruktur ohne Signalsequenz aus der Aminosäuresequenz des Ferritins 2 von *Rhipicephalus microplus* (FER2-RM) besteht.

5. Ferritin 2 für die Immunisierung des Körpers gegen Zecken nach Anspruch 1, dessen Primärstruktur ohne Signalsequenz aus der Aminosäuresequenz des Ferritins 2 von *Dermacentor variabilis* (FER2-DV) besteht.

## Revendications

1. Ferritine 2 pour l'immunisation de l'hôte contre les tiques qui a la séquence d'acides aminés sans la séquence signal dans au moins 85% identique à la séquence d'acides aminés de la ferritine 2 d'*Ixodes ricinus* (FER2-IR) 
GNNLFENLDKYPLQDECQAALQEHINVEMHASLVYMQMAAHFDNNKVARKGFSTFFAENSK
EEREHAQKIIDYINKRGSTVSLVNIDMPLITTWKSVLQALRDAISLENKVTNKLHAVHKIA
DEECKDPQLMDFIESEFLEEQVNSIDKLQRMITVLSNMDSGTGEYLLDRELLGDKKEF
 ou la ferritine 2 d'*Ixodes scapularis* (FER2-IS) 
GNNLFENLDKYPLQDECQAALQEHINVEMHASLVYMQMAAHFDNNKVARKGFSTFFAENSK
EEREHAQKIIDYINKRGSTVSLVNIDMPQITTWKSVLQALRDAISLENKVTNKLHAVHKTA
DEECKDPQLMDFIESEFLEEQVTSIDKLQRMITVLSNMDSGTGEYLLDRELLGDKKEF
 ou la ferritine 2 de *Rhipicephalus microplus* (FER2-RM) 
GNNLNDQVNKYILPDRCRAGLQEQLNLELHASLVYMQMAAHLANNKVARGGFARFFRDQSS
EEREHAQKIIDYLNLRGGTVSAVNVDMPPTAIWMSVLDALQAALALEHRVTNRLYELHRLA
EEYDAQMADFLEQEFLAEQVRSIDQLQRLITQLQNMETGLGEFLLDQQLRA
 ou la ferritine 2 de *Dermacentor variabilis* (FER2-DV) 
GNNLNEQVNQNRYFLHDRCRIGLQEQVNLELHASLVYMQMAAHLANNKVARNGFARFFRDQ
SSEEREHAQKLVDYVNLRGGTVSAVSVDMPATATWMSVLDALQAALALEHRVTNRLHELHR
LADDSQDPQMADFLEQEFLAEQVRSIDQLQRLITQLQNMDTGLGEFLLDQQLRA
 et dans les conditions natives, elle forme la structure quaternaire, qui transporte le fer non hémique de l'intestin de la tique dans les tissus périphériques.

2. Ferritine 2 pour l'immunisation de l'hôte contre les tiques selon la revendication 1, qui a la structure primaire sans séquence signal composée d'une séquence d'acides aminés de la ferritine 2 d'*Ixodes ricinus* (FER2-IR).

3. Ferritine 2 pour l'immunisation de l'hôte contre les tiques selon la revendication 1, qui a la structure primaire sans séquence signal composée d'une séquence d'acides aminés de la ferritine 2 d'*Ixodes scapularis* (FER2-IS).

4. Ferritine 2 pour l'immunisation de l'hôte contre les tiques selon la revendication 1, qui a la structure primaire sans séquence signal composée d'une séquence d'acides aminés de la ferritine 2 de *Rhipicephalus microplus* (FER2-RM).

5. Ferritine 2 pour l'immunisation de l'hôte contre les tiques selon la revendication 1, qui a la structure primaire sans séquence signal composée d'une séquence d'acides aminés de la ferritine 2 de *Dermacentor variabilis* (FER2-DV).
